# EUROPEAN PATENT SPECIFICATION

(11) **EP 3 927 729 B1**
(45) Date of publication and mention of the grant of the patent: **07.10.2026**
(21) Application number: 20758503.5
(22) Date of filing: 11.02.2020
(51) Int. Cl.: C07K 14/54, A61K 9/00, A61K 9/08, A61K 38/19, A61K 38/20, A61K 39/395, A61K 47/02, A61K 47/12, A61K 47/18, A61K 47/26, C07K 16/24, C07K 16/28, C07K 16/46

(54) **THERAPEUTIC ANTI-IL17A ANTIBODY FORMULATION**
THERAPEUTISCHE ANTI-IL17A-ANTIKÖRPERFORMULIERUNG
FORMULATION THERAPEUTIQUE D' ANTI-IL17A ANTICORPS

(30) Priority: 18.02.2019 US 201962807006 P; 31.07.2019 US 201962880846 P; 12.12.2019 US 201962947198 P
(43) Date of publication of application: 29.12.2021
(73) Proprietor: Eli Lilly and Company, Indianapolis, IN 46285 (US)
(72) Inventor: CORVARI, Vincent John, Indianapolis, Indiana 46206-6288 (US); PISUPATI, Karthik, Indianapolis, Indiana 46206-6288 (US); SHI, Galen Huaiqiu, Indianapolis, Indiana 46206-6288 (US)
(74) Representative: Eli Lilly and Company Limited
(86) International application number: PCT/US2020/017594
(87) International publication number: WO 2020/172002

(56) References cited:
- WO-A1-2016/059512
- CN-A- 106 474 470
- US-A1- 2012 315 285
- US-A1- 2014 348 856
- US-A1- 2017 218 092
- US-A1- 2018 201 673
- US-B2- 9 700 485

## Description

The present invention is in the field of medicine. More particularly, the present invention relates to aqueous pharmaceutical formulations comprising therapeutic antibodies that are suitable for subcutaneous ("SQ"), intramuscular ("IM"), and/or intraperitoneal ("IP") administration.

Administration of therapeutic antibodies via SQ, IP and/or IM administration is both common and advantageous. Such routes of administration allow the therapeutic antibody to be delivered in a short period of time and allow patients to self-administer therapeutic antibodies without visiting a medical practitioner. However, formulating therapeutic antibodies into aqueous pharmaceutical formulations suitable for SQ, IM and/or IP administration is both challenging and unpredictable. Additionally, undesirable injection-associated pain, even after a syringe needle is removed, has been reported with such routes of administration and can impair patient compliance with therapy.

The challenge and unpredictability associated with formulating therapeutic antibodies into aqueous pharmaceutical formulations suitable for SQ, IM and/or IP administration is due, in part, to the numerous properties a pharmaceutical formulation must possess to be therapeutically viable. Aqueous pharmaceutical formulations must provide stability to the therapeutic antibody in solution while, at the same time, maintaining the therapeutic antibody's functional characteristics essential for therapeutic efficacy such as target affinity, selectivity and potency. In addition, the aqueous pharmaceutical formulation must also be safe for administration to, and well tolerated by, patients as well as being suitable for manufacturing and storage.

Formulating high concentrations of therapeutic antibodies is even more complex. For example, increased rates of antibody degradation, cleavage, clipping, high molecular weight aggregation, dimerization, trimerization, precipitation pH shift, turbidity, solution color change, changes in charge, isomerization, oxidation and/or deamination (all of which affect the therapeutic antibody concentration, functionality and efficacy) have been reported for aqueous formulations of highly concentrated therapeutic antibodies. Another known challenge when formulating high concentrations of therapeutic antibodies is an increase in viscosity which can negatively affect SQ, IM and/or IP administration of an aqueous pharmaceutical formulation. Additionally, injection-associated pain has been reported with formulations having increased viscosity.

Furthermore, some therapeutic antibodies such as ixekizumab possess charge distributions leading to high levels of intermolecular interactions (e.g., as may be shown by Dynamic Light Scattering), phase separation, gelation and precipitation, making solubility of the molecule in aqueous solution, especially at high concentrations, very challenging to balance. Charge distribution of such antibodies may also manifest in an isoelectric point preventing formulation at neutral pH. For example, some therapeutic antibodies have a polarity, or dipole moment, such that they are only stable in aqueous formulations within narrow, non-neutral, pH windows. Injection-associated pain has been reported, however, for acidic (e.g., ≤ pH 6.5) pharmaceutical formulations of therapeutic antibodies. Thus, such therapeutic antibodies, such as ixekizumab which possesses an isoelectric point of 8.1 (requiring acidic pH formulation), pose additional, unpredictable challenges for formulating in a way that balances stability of the therapeutic antibody with functional properties required for efficacy, as well as tolerability by patients.

Ixekizumab is a highly specific anti-IL17A antagonistic antibody, as described, for example, in U.S. Patent Number 7,838,638. Commercially marketed under the tradename TALTZ^{®}, ixekizumab is administered subcutaneously to patients in a highly concentrated (about 80 mg/mL) pharmaceutical formulation having an acidic pH (about 5.7). The commercial pharmaceutical formulation of ixekizumab, as described in U.S. Patent Number 9,376,491, also includes high concentrations of citrate buffer (about 20mM) and NaCl (about 200mM). However, pharmaceutical formulations having acidic pH and high concentrations of NaCl and/or citrate buffer have been associated with injection-associated pain and patients have reported injection-associated pain after injecting the commercial pharmaceutical formulation of ixekizumab. CN 106 474 470 and WO 2016/059512 disclose anti-IL-17A antibody containing formulations.

Injection-associated pain of aqueous pharmaceutical formulations comprising therapeutic antibodies is a complex, multifactorial issue. For example, each individual component, and/or concentration, ratio and characteristic thereof, of an aqueous pharmaceutical formulation can impact injection-associated pain associated with a therapeutic. Likewise, individual components (and/or concentrations, ratios and characteristics thereof) can impact the stability, functional characteristics, manufacturability and/or tolerability of a formulated therapeutic antibody in an aqueous pharmaceutical formulation. Thus, while a specific formulation adjustment may provide a beneficial impact to a given aspect of the formulation, the same adjustment may also negatively impact other aspects of the formulation. Even further adding to the complexity, a nearly limitless number of different formulation components (e.g., buffers and excipients), as well as concentrations and ratios thereof, have been reported. However, there remains little-to-no correlation for predicting the impact of a specific formulation on the various properties and characteristics of a given therapeutic antibody.

Accordingly, there is a need for an aqueous pharmaceutical formulation of therapeutic antibodies suitable for SQ, IM and/or IP administration and which is well tolerated by patients, exhibiting a therapeutically beneficial level of injection-associated pain. More particularly, there is a need for such aqueous pharmaceutical formulation for highly concentrated therapeutic antibodies possessing an isoelectric point not compatible with neutral pH in solution, requiring aqueous formulation at an acidic pH. Even more particularly, there is a need for an aqueous pharmaceutical formulation of ixekizumab suitable for SQ, IM and/or IP administration and which is well tolerated by patients, exhibiting an improved level of injection-associated pain over the commercial pharmaceutical formulation of ixekizumab (as described in U.S. Patent Number 9,376,491). Such aqueous pharmaceutical formulation must also provide stability for the therapeutic antibody and preserve the properties of the therapeutic antibody essential for therapeutic efficacy. Such aqueous pharmaceutical formulations must also be amendable to manufacturing, preferably having an extended shelf life.

The aqueous pharmaceutical formulations provided herein satisfy the aforementioned needs in a surprising and unexpected way. More particularly, the aqueous pharmaceutical formulations provided herein are bufferless aqueous pharmaceutical formulations, suitable for SQ, IM and/or IP administration of high concentrations of ixekizumab, while also preserving the functional characteristics of ixekizumab essential for therapeutic efficacy. Additionally, the aqueous pharmaceutical formulations provided herein are well tolerated by patients, exhibiting an improved level of injection-associated pain over the commercial pharmaceutical formulation of ixekizumab and providing a therapeutically favorable level of injection-associated pain.

Accordingly, the present disclosure provides a bufferless, aqueous pharmaceutical formulation for administering SQ, IM or IP a high concentration of a therapeutic antibody to a patient with a therapeutically favorable level of injection-associated pain, the aqueous pharmaceutical formulation comprising a therapeutic antibody at a concentration of greater than 50mg/mL, 60mg/mL, 70mg/mL, 80 mg/mL, 90mg/mL, 100mg/mL, 110mg/mL or 120mg/mL; sucrose in a concentration of 234mM +/- 10%; and a surfactant in a concentration between 0.005% w/v +/- 10% to 0.05% w/v +/- 10%, wherein, the pharmaceutical formulation is an aqueous solution at a pH between 5.2 to 6.5. According to specific embodiments, the surfactant is polysorbate 20 or polysorbate 80. In further specific embodiments, the surfactant is polysorbate 80. According to some embodiments, the bufferless aqueous pharmaceutical formulation is substantially free of an ionic tonicity excipient. In some embodiments, the pharmaceutical formulation is substantially free of L-amino acid excipients. In further embodiments, the antibody possesses an isoelectric point not compatible with neutral pH in solution. In some such embodiments, the antibody possesses an isoelectric point of ≥ 7.5 and in even further embodiments, the antibody possesses an isoelectric point of ≥ 8.0. In further, specific embodiments of the aqueous pharmaceutical formulations provided herein, the therapeutic antibody is an anti-IL-17A antibody comprising a LCVR having the amino acid sequence of SEQ ID NO.7 and a HCVR having the amino acid sequence of SEQ ID NO.8. In even further specific embodiments, the anti-IL17A antibody comprises a light chain (LC) having the amino acid sequence of SEQ ID NO.9 and a heavy chain (HC) having the amino acid sequence of SEQ ID NO.10. According to embodiments of the present disclosure, an aqueous pharmaceutical formulation of the present disclosure is provided, wherein the aqueous pharmaceutical formulation upon SQ, IP and/or IM administration to a patient exhibits a reduced risk of, and/or a, therapeutically favorable level of injection-associated pain.

According to particular embodiments of the present disclosure, a bufferless aqueous pharmaceutical formulation for an anti-IL7A antibody is provided. In embodiments, the anti-IL7A antibody comprises a light chain variable region (LCVR) comprising complementarity determining regions (CDRs) LCDR1, LCDR2, and LCDR3 and a heavy chain variable region (HCVR) comprising CDRs HCDR1, HCDR2, and HCDR3, wherein LCDR1 has the amino acid sequence of SEQ ID NO.1, LCDR2 has the amino acid sequence of SEQ ID NO.2, LCDR3 has the amino acid sequence of SEQ ID NO.3, HCDR1 has the amino acid sequence of SEQ ID NO.4, HCDR2 has the amino acid sequence of SEQ ID NO.5, and HCDR3 has the amino acid sequence of SEQ ID NO.6. According to such embodiments, the aqueous pharmaceutical formulation is an aqueous solution at a pH of between 5.2 to 6.5, and comprises the anti-IL17A antibody in a concentration of greater than 60 mg/mL +/- 10%,70 mg/mL +/- 10%, 80 mg/mL +/- 10%, 88 mg/mL +/- 10%, 100 mg/mL +/- 10%, 120 mg/mL +/- 10% or 160 mg/mL +/- 10%; sucrose in a concentration of 234mM +/- 10%; and a surfactant in a concentration of 0.005 +/- 10% to 0.05 +/- 10% %w/v. According to some embodiments, the bufferless aqueous pharmaceutical formulation is substantially free of an ionic tonicity excipient. In some embodiments, the pharmaceutical formulation is substantially free of L-amino acid excipients. In some embodiments, the surfactant is one of polysorbate 20 or 80. In more specific embodiments, the surfactant is polysorbate 80. In even more specific embodiments, the polysorbate 80 is at a concentration of 0.03% w/v +/- 10%. According to such embodiments, the bufferless aqueous pharmaceutical formulation is suitable for SQ, IP and/or IM administration to a patient and exhibits an improved level of injection-associated pain over the commercial pharmaceutical formulation of ixekizumab and/or provides a therapeutically favorable level of injection-associated pain.

In particular embodiments, the aqueous pharmaceutical formulations provided herein comprise an antibody in a concentration of about 80 mg/mL (e.g., +/- 10%); sucrose in a concentration of about 234 mM (e.g., +/- 10%); and polysorbate 80 in a concentration of about 0.03 %w/v (e.g., +/- 10%), and the pharmaceutical formulation is substantially free of an ionic tonicity excipient, substantially free of L-amino acid excipients, and is at a pH of about 5.7 (e.g., +/- 10%), and the antibody is an anti-IL17A antibody comprising a LCVR having the amino acid sequence of SEQ ID NO.7 and a HCVR having the amino acid sequence of SEQ ID NO.8. In further such embodiments, the anti-IL17A antibody comprising a heavy chain having the amino acid sequence of SEQ ID NO. 10 and a light chain having the amino acid sequence of SEQ ID NO. 9. According to such embodiments, the aqueous pharmaceutical formulation is suitable for SQ, IP and/or IM administration to a patient and exhibits an improved level of injection-associated pain over the commercial pharmaceutical formulation of ixekizumab and/or provides a therapeutically favorable level of injection-associated pain.

In further embodiments, a system for subcutaneously delivering an aqueous pharmaceutical formulation to a patient in need of treatment is provided. Such system includes a device having a chamber, a drive mechanism operatively coupled to the chamber, and a needle, the chamber being capable of storing a liquid, the needle having a bore in fluid communication with an outlet of the chamber to receive a liquid from the chamber, and the drive mechanism being operative to force the transfer of a liquid from the chamber into the bore of the needle. Such system also includes a pharmaceutical formulation of the present disclosure disposed within the chamber and the inner wall of the chamber having a silicone oil coating at an amount of less than about 0.4 mg. According to some more specific embodiments, the inner wall of the chamber has a silicone oil coating at an amount of about 0.2 mg or an amount of less than about 0.2mg. According to some embodiments of the system, the patient is in need of treatment of RA, Ps, GenPs, Pruritus, AS, PA, PPP, HS or MM.

In further embodiments, the present disclosure provides a method for reducing injection-associated pain and/or providing a therapeutically favorable level of injection-associated pain experienced by a patient at the time of, or shortly after, SQ, IM and/or IP injection of an aqueous pharmaceutical formulation comprising a therapeutic antibody, the method comprising administering to a patient an aqueous pharmaceutical formulation of the present disclosure. According to embodiments, the present disclosure provides a method of delivering a therapeutic antibody to a patient with a therapeutically favorable level of injection-associated pain, wherein the method comprises administering to a patient a pharmaceutical formulation of the present disclosure, wherein the method provides a therapeutically favorable level of injection-associated pain. According to further embodiments, the present disclosure provides an improved method of delivering a therapeutic antibody to a patient, wherein the improvement comprises a reduction in, and/or providing a therapeutically favorable level of, injection-associated pain with SQ, IM or IP administration of an aqueous pharmaceutical formulation, the method comprising administering to a patient an aqueous pharmaceutical formulation of the present disclosure. According to embodiments, the reduction in injection-associated pain comprises a reduction from commercially available formulations and/or providing a therapeutically favorable level of injection-associated pain. According to embodiments, a therapeutically favorable level of injection-associated pain may comprise a VAS score of less than 30mm or a VAS score of less than 20mm.

According to embodiments, the present disclosure provides an improved method for administering an anti-IL17A antibody to a patient in need thereof, wherein the improvement comprises a reduction in the level of injection-associated pain upon the administration of a SQ, IM or IP injection of an aqueous pharmaceutical formulation, the method comprising administering to the patient an aqueous pharmaceutical formulation of the present disclosure, wherein said step of administering provides an improved level of injection-associated pain and/or provides a therapeutically favorable level of injection-associated pain. According to some embodiments, the aqueous pharmaceutical formulation consists essentially of an aqueous pharmaceutical formulation of the present disclosure. According to embodiments, the reduction in the level of injection-associated pain comprises providing an improved level of injection-associated pain (for example, a reduction in VAS score compared to the commercial formulation of ixekizumab, i.e., the citrate and NaCl formulation exemplified by the control formulation of Table 2). According to some embodiments, the method provides a therapeutically favorable level of injection-associated pain comprising a VAS score of less than 30 mm or less than 20 mm. According to embodiments, the anti-IL17A antibody is ixekizumab and, according to some such embodiments, the improved level of injection-associated pain comprises a reduction in VAS score compared to the commercial formulation of ixekizumab (the citrate and NaCl formulation exemplified by the control formulation of Table 2). According to some embodiments, the aqueous pharmaceutical formulation is administered by SQ injection.

According to further embodiments of the present disclosure, an improved method of treating at least one of PsO, PsA and AxSpa is provided, wherein the improvement comprises a reduction in injection-associated pain upon the SQ administration of an aqueous pharmaceutical formulation comprising an anti-IL17A antibody, the method comprising administering an aqueous pharmaceutical formulation of the present disclosure, wherein said step of administering provides an improved level of injection-associated pain and/or provides a therapeutically favorable level of injection-associated pain. According to some embodiments, a therapeutically favorable level of injection-associated pain is provided comprising a VAS score of less than 30 mm or less than 20 mm. In some more specific embodiments, the anti-IL17A antibody is ixekizumab and, according to some such embodiments, the improved level of injection-associated pain comprises a reduction in VAS score compared to the commercial formulation of ixekizumab (the citrate and NaCl formulation exemplified by the control formulation of Table 2).

The present disclosure also provides an aqueous pharmaceutical formulation of the present disclosure for use in therapy. In particular embodiments, the present disclosure provides an aqueous pharmaceutical formulation of the present disclosure for use in the treatment of rheumatoid arthritis (RA), psoriasis (Ps), genital psoriasis (GenPs), pruritus, ankylosing spondylitis (AS), psoriatic arthritis (PA), palmoplantar pustulosis (PPP), Hidradenitis suppurativa (HS) or multiple myeloma (MM). According to further embodiments of the present disclosure, a use of an aqueous pharmaceutical formulation of the present disclosure for the manufacturer of a medicament for the treatment of RA, Ps, GenPs, pruritus, AS, PA, PPP, HS or MM is provided. According to such embodiments, use of such aqueous pharmaceutical formulations is suitable for SQ, IP and/or IM administration to a patient and exhibits an improved level of injection-associated pain over the commercial pharmaceutical formulation of ixekizumab and/or provides a therapeutically favorable level of injection-associated pain.

According to particular embodiments, the present disclosure provides a method of treating RA, Ps, GenPs, pruritus, AS, PA, PPP, HS or MM comprising administering to a patient in need thereof an effective amount of an aqueous pharmaceutical formulation of the present disclosure, wherein the aqueous pharmaceutical formulation comprises an anti-IL17A antibody. In a more particular embodiment, such method of treating includes administering subcutaneously, to the patient, an initial dose of the aqueous pharmaceutical formulation, on day 0, followed by administering subcutaneously the aqueous pharmaceutical formulation to the patient at every four week interval thereafter, wherein the aqueous pharmaceutical formulation administered to the patient at every four week interval after the initial dose comprises the anti-IL17A antibody at a concentration of about 80 mg/mL. In another particular embodiment, such method of treating includes administering subcutaneously, to the patient, an initial dose of the aqueous pharmaceutical formulation, on day 0, followed by administering subcutaneously the aqueous pharmaceutical formulation to the patient at every two week interval thereafter, wherein the aqueous pharmaceutical formulation administered to the patient at every two week interval after the initial dose comprises the anti-IL17A antibody at a concentration of about 80 mg/mL. In yet another particular embodiment, such method of treating includes administering subcutaneously, to the patient, an initial dose of the aqueous pharmaceutical formulation, on day 0, followed by administering subcutaneously the aqueous pharmaceutical formulation to the patient on each of days 14, 28, 42, 56, 70 and 84, and followed by administering subcutaneously the aqueous pharmaceutical formulation to the patient at every four week interval thereafter, wherein the aqueous pharmaceutical formulation, administered to the patient at each of days 14, 28, 42, 56, 70 and 84, and every four week interval thereafter, comprises the anti-IL17A antibody at a concentration of about 80 mg/mL. According to some of the methods of treating provided by the instant disclosure, the initial dose of the aqueous pharmaceutical formulation comprises about 160 mg of the anti-IL17A antibody. In some such embodiments, the about 160 mg initial dose of the aqueous pharmaceutical formulation comprises two doses of the aqueous pharmaceutical formulation, each dose comprising about 80 mg of the anti-IL17A antibody. According to such methods, the aqueous pharmaceutical formulation exhibits an improved level of injection-associated pain over the commercial pharmaceutical formulation of ixekizumab and/or provides a therapeutically favorable level of injection-associated pain.

According to particular embodiments, there is provided herein an aqueous pharmaceutical formulation comprising an anti-IL17A antibody for use in the treatment of RA, Ps, GenPs, pruritus, AS, PA, PPP, HS or MM wherein the pharmaceutical formulation is to be administered subcutaneously with an initial dose on day 0, followed by a dose every four weeks interval thereafter, wherein the pharmaceutical formulation to be administered at every four week interval after the initial dose comprises the anti-IL17A antibody at a concentration of about 80 mg/mL. In another particular embodiment, there is provided pharmaceutical formulations disclosed herein comprising an anti-IL17A antibody for use in the treatment of RA, Ps, GenPs, pruritus, AS, PA, PPP, HS or MM wherein the pharmaceutical formulation is to be administered subcutaneously with an initial dose on day 0, followed by a dose every two weeks interval thereafter, wherein the pharmaceutical formulation to be administered at every two week interval after the initial dose comprises the anti-IL17A antibody at a concentration of about 80 mg/mL. In yet another particular embodiment, there is provided pharmaceutical formulations disclosed herein comprising an anti-IL17A antibody for use in the treatment of RA, Ps, GenPs, pruritus, AS, PA, PPP, HS or MM wherein the pharmaceutical formulation is to be administered subcutaneously with an initial dose on day 0, followed by a dose on each of days 14, 28, 42, 56, 70 and 84, wherein the pharmaceutical formulation to be administered on each of days 14, 28, 42, 56, 70 and 84 after the initial dose comprises the anti-IL17A antibody at a concentration of about 80 mg/mL. According to some embodiments, the initial dose of the aqueous pharmaceutical formulation comprises about 160 mg of the anti-IL17A antibody. In some such embodiments, the about 160 mg initial dose of the aqueous pharmaceutical formulation comprises two doses of the aqueous pharmaceutical formulation, each dose comprising about 80 mg of the anti-IL17A antibody. According to such embodiments, the aqueous pharmaceutical formulations provided herein exhibit an improved level of injection-associated pain over the commercial pharmaceutical formulation of ixekizumab and/or provide a therapeutically favorable level of injection-associated pain.

As used interchangeably herein, the expressions "aqueous pharmaceutical formulation" or "pharmaceutical formulation" mean an aqueous solution having at least one therapeutic antibody capable of exerting a biological effect in a human, at least one inactive ingredient (e.g., excipient, surfactant, etc.) which, when combined with the therapeutic antibody, is suitable for therapeutic administration to a human. The pharmaceutical formulations provided by the present disclosure are bufferless (i.e., do not comprise agents such as citrate buffer, histidine buffer, acetate buffer, or the like, or combinations thereof, which have acid-base conjugate components, for resisting pH change), aqueous, stable formulations wherein the degree of degradation, modification, aggregation, loss of biological activity and the like, of therapeutic antibodies therein, is acceptably controlled and does not increase unacceptably with time.

As used herein, the term "antibody" refers to an immunoglobulin G (IgG) molecule comprising two heavy chains ("HC") and two light chains ("LC") interconnected by disulfide bonds. Each heavy chain is comprised of a heavy chain variable region ("HCVR") and a heavy chain constant region ("CH"). Each light chain is comprised of a light chain variable region ("LCVR") and a light chain constant region ("CL"). Each HCVR and LCVR are further sub-dividable into regions of hypervariability, termed complementarity determining regions ("CDR"), interspersed with regions that are more conserved, termed framework regions ("FR"). Each HCVR and LCVR is composed of three CDRs and four FRs arranged from amino-terminus to carboxy-terminus in the following order: FR1, CDR1, FR2, CDR2, FR3, CDR3, FR4. The variable regions of each HC and LC contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

According to particular embodiments of aqueous pharmaceutical formulations provided herein, the antibodies are anti-IL17A antibodies. Interleukin 17A, or IL17A, as used herein refers to cytokines of the IL17 cytokine family (also known as cytotoxic T-lymphocyte-associated antigen 8 ("CTLA8")). IL17A cytokines exist as homodimeric complexes (e.g., IL17A/A) or as heterodimeric complexes in complex with another IL17 cytokine family member such as IL17F (e.g., IL17A/F). IL17A cytokines are believed to be produced primarily by effector T helper (Th17) cells and have been shown to induce secretion of pro-inflammatory cytokines such as IL-6, IL-8, IL-1 and TNF. The homodimeric complex form of IL17A, IL17A/A, has been shown to play a role in diseases such as psoriasis and psoriatic arthritis, both immune-related diseases associated with T cell dysregulation.

When referred to herein, such anti-IL17A antibodies are antibodies that specifically bind and antagonize human IL17A by way of specificity for the A subunit (e.g., the A subunit of IL17A/F or one or both of the A subunits of IL17A/A). According to specific embodiments of anti-IL17A antibodies, LCDR1 comprises the amino acid sequence of SEQ ID NO.1, LCDR2 comprises the amino acid sequence of SEQ ID NO.2, LCDR3 comprises the amino acid sequence of SEQ ID NO.3, HCDR1 comprises the amino acid sequence of SEQ ID NO.4, HCDR2 comprises the amino acid sequence of SEQ ID NO.5, and HCDR3 comprises the amino acid sequence of SEQ ID NO.6. According to some such embodiments, the LCVR comprises the amino acid sequence of SEQ ID NO.7 and the HCVR comprises the amino acid sequence of SEQ ID NO.8. In even more specific embodiments of such anti-IL17 antibodies, the LC comprises the amino acid sequence of SEQ ID NO.9 and the HC comprises the amino acid sequence of SEQ ID NO.10. An exemplary embodiment of an anti-IL17A antibody is ixekizumab, as described, for example, in U.S. Patent Number 7,838,638. An additional example of an anti-IL17A antibody is secukinumab (marketed under the tradename COSENTYX^{®}), as described, for example, in U.S. Patent Number 7,807,155.

As may be used herein, the terms "about" or "approximately", when used in reference to a particular recited numerical value or range of values, means that the value may vary from the recited value by no more than 10% (e.g., +/- 10%). For example, as used herein, the expression "about 100" includes 90 and 110 and all values in between (e.g., 91, 92, 93, 94, etc.).

As referred to herein, the terms "substantially free of" or "substantially devoid of" mean the presence of a given substance (e.g., ionic tonicity excipient) is below a limitation of detection for an assay used for detecting the presence of such substance.

The term "ionic tonicity excipient", as referred to herein, means an excipient that comprises an ionic compound (e.g., an electrolyte such as sodium chloride, potassium chloride, magnesium chloride, calcium chloride, arginine hydrochloride, or the like), which is distinct from the antibody and surfactant comprising an aqueous pharmaceutical formulation. An ionic tonicity excipient, as is known in the field, may be used to adjust the osmotic pressure of a pharmaceutical formulation. However (and as provided in the examples provided herein), adjustment of pH with HCl or NaOH, as necessary, following dissolution and mixing of the aqueous pharmaceutical formulation is not within the meaning of the term ionic tonicity excipient as used herein (as HCl or NaOH, added for pH adjustment are not acting in the formulation as an ionic tonicity excipient).

As referred to herein, the term L-amino acid excipients refers to L-amino acids which are added as either a part of a buffer (e.g., L-histidine in a histidine buffer; L-arginine in an arginine buffer, etc.) or as an excipient component of an aqueous pharmaceutical formulation (but does not refer to components of the therapeutic antibody).

As referred to interchangeably herein, the "visual analog scale" or "VAS", refers to an evaluation tool for assessing injection-associated pain experienced by a patient. VAS consists of a 100 mm contiguous scale, upon which a patient identifies their level of pain following injection. The VAS scoring extremes are "no pain at all" (e.g., 0) and "worst pain imaginable" (e.g., 100). Severity of pain may be categorized, according to the VAS tool, as mild pain (≤30 mm); moderate pain (>30 mm - ≤70 mm) and severe pain (>70 mm). When referred to herein, "injection-associated pain" is in reference to acute pain experienced by a patient at the time of, or shortly after, injection of an aqueous pharmaceutical formulation. A desired property of a stable pharmaceutical formulation is being well tolerated by patients, for example, providing a therapeutically favorable level of injection-associated pain (e.g., a VAS score of <30 mm and/or <20 mm). As is known, the components, and concentrations and/or ratios thereof, of a pharmaceutical formulation may impact injection-associated pain experienced by the patient.

As used interchangeably herein, "treatment" and/or "treating" and/or "treat" are intended to refer to all processes wherein there may be a total elimination, slowing or delaying, reduction in severity or frequency (e.g., of flares or episodes), interruption or stopping of the progression of disease and/or symptoms thereof, but does not require a total elimination of all disease symptoms. Treatment includes administration of an aqueous pharmaceutical formulation of the present disclosure for treatment of a disease in a human that would benefit from at least one of the above-listed processes, including: (a) inhibiting further progression of disease symptoms and effects, *i.e.,* arresting its development; (b) relieving the disease, i.e., causing an elimination or regression of disease, disease symptoms or complications thereof; and (c) preventing or reducing the frequency of disease episodes or flares. According to specific embodiments, the pharmaceutical formulations provided herein may be used in the treatment of at least one of RA, Ps, GenPs, AS, PA, PPP, HS or MM.

As used interchangeably herein, the term "patient," "subject" and "individual," refers to a human. Unless otherwise noted, the subject is further characterized as having, being at risk of developing, or experiencing symptoms of a disease that would benefit from administration of a pharmaceutical formulation disclosed herein.

As used interchangeably herein, an "effective amount" or "therapeutically effective amount" of a pharmaceutical formulation of the instant disclosure refers to an amount necessary (at dosages, frequency of administration and for periods of time for a particular means of administration) to achieve the desired therapeutic result. An effective amount of pharmaceutical formulation of the present disclosure may vary according to factors such as the disease state, age, sex, and weight of the subject and the ability of the pharmaceutical formulation of the present disclosure to elicit a desired response in the subject. An effective amount is also one in which any toxic or detrimental effects of the pharmaceutical formulation of the present disclosure are outweighed by the therapeutically beneficial effects.

The instant disclosure also relates to dose regimens for the treatment of a disease with a pharmaceutical formulation of the present disclosure. As referred to herein and as generally known in the art, the term "dose" refers to an amount of a pharmaceutical formulation that is administered to a subject. A "dose regimen" or "dosage regimen", as generally known in the field and as may be referred to interchangeably herein, includes a treatment schedule for administering a set (i.e., series or sequence) of doses to be administered to a patient over a period of time.

By way of example, a dose regimen of the present disclosure may include an initial dose of an aqueous pharmaceutical formulation (for example, comprising an anti-IL17A antibody) of the present disclosure administered to a patient on the first day of treatment (e.g., Day 0). An initial dose may be referred to herein as a "loading dose". Additionally, a dose regimen of the present disclosure may include an initial period of treatment, sometimes referred to herein as an "induction period", which follows the loading dose. During an induction period, for example, a patient may be administered a dose (or doses) comprising a specific concentration of a therapeutic antibody (e.g., anti-IL17A antibody), at a given frequency of administration (e.g., every day, every 2 weeks, every 4 weeks, etc.), for a given duration of time (e.g., 4, 12 or 16 weeks). Additionally, dose regimens of the present disclosure may include a period following the induction period, sometimes referred to herein as the "maintenance period", in which a patient is administered a dose comprising a specific concentration of the therapeutic antibody, at a given frequency of administration (e.g., every 2 or 4 weeks, etc.).

The aqueous pharmaceutical formulations of the present disclosure may be administered to a patient via parenteral administration. Parenteral administration, as understood in the medical field, refers to the injection of a dose into the body by a sterile syringe or some other drug delivery system including an autoinjector or an infusion pump. Exemplary drug delivery systems for use with the aqueous pharmaceutical formulations of the present disclosure are described in the following references: U.S. Patent Publication No. 2014/0054883 to Lanigan et al., filed March 7, 2013 and entitled "Infusion Pump Assembly"; U.S. Patent No. 7,291,132 to DeRuntz et al., filed February 3, 2006 and entitled "Medication Dispensing Apparatus with Triple Screw Threads for Mechanical Advantage"; U.S. Patent No. 7,517,334 to Jacobs et al., filed September 18, 2006 and entitled "Medication Dispensing Apparatus with Spring-Driven Locking Feature Enabled by Administration of Final Dose"; and U.S. Patent No. 8,734,394 to Adams et al., filed August 24, 2012 and entitled "Automatic Injection Device with Delay Mechanism Including Dual Functioning Biasing Member." Parenteral routes include IM, SQ and IP routes of administration.

### Examples

### Exemplary Aqueous Pharmaceutical Formulation

**Table 1. Exemplary Aqueous Pharmaceutical Formulation**

| | **Concentration** |
|---|---|
| anti-IL17A antibody* | 80 mg/mL |
| PS-80 | 0.03% w/v (0.3 mg/mL) |
| Sucrose | 234 mM (8% w/v) |
| pH | 5.7 |

| | |
|---|---|
| * The anti-IL17A antibody comprises an HCVR of SEQ ID NO: 8 and an LCVR of SEQ ID NO: 7. | |

The manufacturing process for the anti-IL17A antibody pharmaceutical formulation presented in Table 1 may be accomplished by weighing an appropriate quantity of water (e.g., at a temperature of 20 +/-5° C) into a tared empty vessel of appropriate size. The appropriate quantity of sucrose is added and mixed. Polysorbate 80 is accurately weighed out in a glass container and an appropriate quantity of water at a temperature of 20 +/-5° C is added into the glass container to give the desired concentration and the solution is mixed. The entire content of the polysorbate 80 solution is added to the other excipients. The polysorbate 80 solution container is rinsed with water to ensure the entire contents are transferred. After addition of the polysorbate 80 solution, the solution is mixed. After dissolution and mixing has been completed, the pH of the solution is checked to be within 5.7 +/-0.3; adjustment with HCl or NaOH solution is done if necessary. The excipient composition is passed through a filter (polyvinylidene fluoride [PVDF]) for bioburden reduction.

The anti-IL17A antibody, previously expressed in cells, purified, and concentrated, is mixed with an appropriate amount of the formulation excipient solution. The pH of the solution is re-checked to be within 5.7+/-0.3. The pharmaceutical formulation is passed through a PVDF filter for bioburden reduction and may then be stored at 5° C.

### Physical-Chemical Properties

Both physical and chemical stability is essential for a pharmaceutical formulation of a therapeutic antibody to allow storage and transportation (e.g., 1 year, 18 months, or 2 years) and preserve safety and efficacy. Exemplary evaluations to gauge the physical stability of a pharmaceutical formulation include solubility (phase-separation, gelation) assessments, molecular interactions (e.g., as measured by DLS), visual clarity (i.e., opalescence) characterization by turbidity assessment, and viscosity measurement. Additionally, chemical stability may be assessed using various analytical methods including size exclusion chromatography (SEC), cation exchange chromatography (CEX) HPLC, reduced and non-reduced capillary electrophoresis (CE-SDS R/NR) and particulate analysis. As demonstrated herein, the exemplified anti-IL17A antibody pharmaceutical formulation of Table 1 demonstrates chemical and physical stability as well as solubility for the highly concentrated therapeutic antibody, ixekizumab, which possesses an isoelectric point of ≥ 7.5, not compatible with formulation at neutral pH in solution.

### Solubility Assessments:

Sufficiently high solubility is essential for an aqueous pharmaceutical formulation. The aqueous pharmaceutical formulation must maintain the antibody in monomeric state, without high molecular weight (HMW) aggregation, at high concentration. Solubility of an anti-IL17A antibody, having an isoelectric point ≥8.0 (in solution), at high concentrations is analyzed under varying conditions.

Samples of each aqueous formulation provided in Table 2 are incubated at each of 5, 0 and -5 degrees Celsius (e.g., samples of each formulation may be incubated, in parallel, at 5, 0 and -5°C) for one week. Following incubation samples are assessed for phase separation, gelation, turbidity and viscosity.

**Table 2: Formulations**

| **Sample ID** | **Buffer** | **Non-Buffer Excipients** | **Anti-IL17A Antibody* Concentration** | **pH** |
|---|---|---|---|---|
| **Control** (commercial formulation as described in U.S. Patent No. 9,376,491) | 20 mM Citrate | 200 mM NaCl | 80 mg/mL | 5.7 |
| | | 0.03% PS-80 | | |
| **1** (formulation of Table 1) | None | 234 mM sucrose | 80 mg/mL | 5.7 |
| | | 0.03%PS-80 | | |
| **2** | 10 mM Citrate | 274 mM mannitol | 80 mg/mL | 5.7 |
| **3** | 10 mM Citrate | 274 mM mannitol | 80 mg/mL | 5.7 |
| | | 0.03% PS80 | | |
| **4** | 10 mM Citrate | 234 mM sucrose | 80 mg/mL | 5.7 |
| | | 0.03%PS-80 | | |
| **5** | 5 mM Citrate | 175 mM NaCl | 80 mg/mL | 5.7 |
| | | 0.03% PS80 | | |
| **6** | 2.69 mM L-histidine | 150 mM NaCl | 80 mg/mL | 6.5 |
| | 6.28 mM L-histidine hydrochloride monohydrate | | | |
| **7** | 2.69 mM L-histidine | 150 mM NaCl | 80 mg/mL | 6.5 |
| | 6.28 mM L-histidine hydrochloride monohydrate | 0.03% PS80 | | |
| **8** | 2.69 mM L-histidine | 150 mM NaCl | 80 mg/mL | 5.7 |
| | 6.28 mM L-histidine hydrochloride monohydrate | 0.03% PS80 | | |
| **9** | None | 130 mM NaCl | 80 mg/mL | 5.7 |
| **10** | None | 100 mM NaCl | 80 mg/mL | 5.7 |
| **11** | None | 65 mM NaCl | 50 mg/mL | 5.7 |
| **12** | 10 mM Citrate | None | 80 mg/mL | 5.7 |

| | | | | |
|---|---|---|---|---|
| *The anti-IL17A antibody comprises two HCVRs having the amino acid sequence of SEQ ID NO: 8 and two LCVRs having the amino acid sequence of SEQ ID NO: 7. **In addition to the tested aqueous pharmaceutical formulations set forth in Table 2, an aqueous pharmaceutical formulation comprising 10 mM acetate buffer, 150 mM NaCl and 80 mg/mL of the anti-IL17A antibody, at pH 5.0, was assessed following incubation, wherein unacceptable levels of antibody clipping were observed by non-reduced CD-SDS. ***Additionally, as set forth in U.S. Patent No. 9,376,491, unacceptable cloud point was observed for the anti-IL17A antibody with concentrations below either of 20 mM citrate buffer and 150 mM NaCl. | | | | |

### Phase Separation:

As detailed in US Patent No. 9,376,491, the exemplified anti-IL17A antibody (comprising two LCVRs having the amino acid sequence of SEQ ID NO: 7 and two HCVRs having the amino acid sequences of SEQ ID NO: 8) has a propensity to phase separate in solution below 0 degrees Celsius (°C). However, storage of drug product is at 5°C and requires stability for periodic refrigeration temperature excursions below 0°C. As provided in U.S. Patent No. 9,376,491, increasing citrate buffer and NaCl concentrations sufficiently lowers the temperature at which phase separation occurs. Injection-associated pain, however, has been reported to be associated with formulations comprising increased citrate buffer and NaCl concentrations and patients have reported injection-associated pain after injecting the commercial pharmaceutical formulation of ixekizumab.

Phase separation of formulations provided in Table 2 is assessed, following incubation at -5°C for one week, by visual monitoring for signs of phase separation (e.g., the formation of a dense, protein rich layer at the bottom of the vial). Results are provided in Table 3.

### Gelation:

Events such as thermodynamic solid phase change (e.g., gelation) can occur at lower temperatures (5°C or lower), negatively impacting stability. As detailed in U.S. Patent No. 9,376,491, gelation has been observed with the exemplified anti-IL17A antibody at high concentrations at temperatures of 5°C and below. U.S. Patent No. 9,376,491 also shows that increasing citrate buffer and NaCl concentration sufficiently avoids gelation at lower temperatures. However, as noted, injection-associated pain has been reported to be associated with formulations comprising increased citrate buffer and NaCl concentrations and patients have reported injection-associated pain after injecting the commercial pharmaceutical formulation of ixekizumab.

Gelation assessment of formulations provided in Table 2 are provided in Table 3. Briefly, following incubation as described above, each vial is agitated (e.g., inverted and then returned upright) and then visually inspected for solidification or lack of liquid flow.

### Turbidity:

Turbidity (i.e., loss of transparency due to particulate matter suspension) is an inherent challenge for aqueous pharmaceutical formulations of therapeutic antibodies. The challenge is exasperated at high concentrations of antibodies and at lower temperatures, which can lead to the formulation failing visual inspection.

Briefly, following incubation as described above, turbidity is assessed (measurements taken at ambient temperature) both visually (e.g., light-based method using purified water as a comparator) and by a nephlometer (HACH Turbidimeter, according to manufacturer instructions) yielding quantitative measurements (NTUs). Lower NTUs are desired; more specifically NTUs values of less than 50 are desired with a failure cut-off at 80 NTUs. Results are provided in Table 3.

### Viscosity:

An aqueous pharmaceutical formulation, to be acceptable for manufacturing, administration to and tolerability by patients must possess appropriate viscosity. Less viscous (at least < 20cP) aqueous solution is required in order to be subcutaneously delivered. Increased concentrations of therapeutic antibody present the challenge of increasing viscosity. It is known that pharmaceutical formulations with NaCl have decreased viscosity, but as noted, increasing NaCl concentration in a pharmaceutical formulation has been associated with injection-associated pain.
Viscosity of formulation 1 and the control formulation of Table 2 is assessed following incubation at 20°C, by viscometer (Anton Paar AMVn Viscometer, according to manufacturer instructions) yielding centipoise (cP) measurements. Lower cP being desired, especially for example, <20cP. Results are provided in Table 3.

**Table 3. Solubility Assessment of the Formulations of Table 2**

| **Sample ID** | **Phase Separation Assessment** | **Gelation Assessment** | **Turbidity (NTUs)** | **Viscosity (cPs)** |
|---|---|---|---|---|
| **Control** | No | No | 63 | 3 |
| **1** | No | No | 10 | 5 |
| **2** | Yes | ND | ND | ND |
| **3** | Yes | ND | ND | ND |
| **4** | Yes | ND | ND | ND |
| **5** | No | No | 85 | ND |
| **6** | No | Yes | ND | ND |
| **7** | No | Yes | ND | ND |
| **8** | No | No | 95 | ND |
| **9** | Yes | ND | ND | ND |
| **10** | Yes | ND | ND | ND |
| **11** | Yes | ND | ND | ND |
| **12** | Yes | ND | ND | ND |

As shown in Table 3, unacceptable phase separation or gelation was observed for all formulations lacking at least 150 mM NaCl (as well as the NaCl bufferless formulations), with the exception of formulation 1 which did not demonstrate phase separation. Phase separation results for formulation 1 are comparable to the control formulation (high citrate, high NaCl formulation). Also, unacceptable gelation was observed for formulations comprising histidine buffer and NaCl at pH 6.5. Formulation 1 did not demonstrate gelation and was comparable to the control formulation (high citrate, high NaCl formulation). Additionally, unacceptable turbidity was observed for both formulation 5 (citrate (5mM), NaCl (175mM)) and formulation 8 (histidine (9mM) and NaCl (150 mM)). Formulation 1 demonstrated acceptable levels of turbidity and provided unexpected improved levels of turbidity compared to the control formulation (high citrate, high NaCl formulation). Further, as shown, both formulation 1 and the control formulation exhibit acceptable and comparable viscosity.

### Chemical Stability:

Chemical stability is essential for the development of an aqueous pharmaceutical formulation both for allowing storage (i.e., sufficient shelf-life) and preserving safety and efficacy. Chemical stability comparing the control and formulation 1 (provided in Table 2) is assessed following an incubation period of four weeks at 25°C or 40°C in accelerated degradation studies. Change in % HMW aggregate is compared against % HMW aggregate at time 0.

In one assessment, the change in high molecular weight (HMW) aggregate in the formulations is assessed using size-exclusion chromatography (SEC) according to standard procedures. Results are provided in Table 4.

**Table 4. Summary of change in % HMW aggregates measured by SEC**

| **Formulation # (of Table 2)** | **Change in %HMW aggregates 25°C** | **Change in %HMW aggregates 40°C** |
|---|---|---|
| Control | 0.25 | 0.05 |
| 1 | 0.49 | 0.43 |

As shown, both the control formulation and formulation 1 of Table 2 demonstrate acceptable and comparable chemical stability in accelerated degradation studies.

Additional accelerated chemical stability of the control and formulation 1 of Table 2 is studied using Cation Exchange (CEX) HPLC. Briefly, samples are incubated at 25°C for four weeks. Following incubation, samples are analyzed for increase in total % acid variants (%AV) using CEX HPLC. Increase in total % acid variants (%AV) provides an indicator of degradation of the therapeutic antibody in the aqueous formulation. Results are provided in Table 5.

**Table 5. Increase in % AV over 4 Weeks at 25°C**

| **Formulation # (of Table 2)** | **Increase in % AV** |
|---|---|
| Control | 2.0 |
| 1 | 2.3 |

As shown, both the control and formulation 1 of Table 2 demonstrate acceptable, and comparable, levels of chemical stability in the further accelerated degradation studies.

### Multivariate Assessment of Formulation 1 of Table 2.

As demonstrated herein, formulation 1 of Table 2 provides unexpected stability comparable to (or improved over) the control formulation of Table 2. A multivariate assessment of physical and chemical stability of formulation 1 of Table 2 is performed as set forth below.

Briefly, four variables (antibody concentration; pH; sucrose concentration; and PS-80 concentration) of formulation 1 of Table 2 are modified to assess physical and chemical stability response of each variable and/or interactions between the variables. Formulation 1 of Table 2 is set as the center point formulation for such experiment. Variant formulations are provided in Table 6.

**Table 6. Variant Formulations**

| **Sample ID** | **Sucrose** | **PS-80**** | **Anti-IL17A* Antibody** | **pH** |
|---|---|---|---|---|
| **Center Point** (formulation 1 of Table 2) | 234 mM | 0.03% | 80 mg/mL | 5.7 |
| **13** | 205 mM | 0.05% | 72 mg/mL | 5.2 |
| **14** | 205 mM | 0.005% | 72 mg/mL | 6.2 |
| **15** | 205 mM | 0.005% | 88 mg/mL | 5.2 |
| **16** | 205 mM | 0.05% | 88 mg/mL | 6.2 |
| **17** | 263 mM | 0.005% | 72 mg/mL | 5.2 |
| **18** | 263 mM | 0.05% | 72 mg/mL | 6.2 |
| **19** | 263 mM | 0.05% | 88 mg/mL | 5.2 |
| **20** | 263 mM | 0.005% | 88 mg/mL | 6.2 |

| | | | | |
|---|---|---|---|---|
| *The anti-IL17A antibody comprises two HCVRs having the amino acid sequence of SEQ ID NO: 8 and two LCVRs having the amino acid sequence of SEQ ID NO: 7. **Polysorbate tolerance for the ranges set forth in Table 6 are confirmed by accelerated freeze-thaw studies. | | | | |

Each variant formulation is assessed for phase separation, gelation and turbidity according to procedures described above. This multivariate assessment provides identification of tolerance limitations for the assessed variables. No phase separation or gelation was observed and acceptable turbidity values were observed.

### Long-term Stability Assessment

Long-term stability of an aqueous pharmaceutical formulation is required to demonstrate storage capability and sufficient shelf life (e.g., 1 year, 2 years or greater). Long-term stability of the center point formulation of Table 6 (which corresponds to the formulation provided in Table 1 and Formulation 1 of Table 2) is assessed following incubation of samples at: 5°C for 1, 3 and 6 months; 25°C for 1 and 3 months; and 35°C for 1 and 3 months (assessment of sample prior to incubation is also performed).

Following incubation, samples are analyzed for percent monomer and percent high molecular weight (HMW) aggregate using size-exclusion chromatography (SEC) according to standard procedures. Results are provided in Table 7.

**Table 7. Long Term Stability Assessment of Center Point Formulation**

| **Incubation Temp (°C)** | **Incubation Period (months)** | **Monomer (%)** | **HMW Aggregate (%)** |
|---|---|---|---|
| **Control (pre-incubation)** | NA | 98.61 | 1.27 |
| 5 | 1 | 98.83 | 1.10 |
| 5 | 3 | 98.57 | 1.39 |
| 5 | 6 | 98.61 | 1.27 |
| 5 | 12 | 98.67 | 1.28 |
| 25 | 1 | 98.59 | 1.32 |
| 25 | 3 | 98.01 | 1.85 |
| 35 | 1 | 97.93 | 1.70 |
| 35 | 3 | 95.54 | 3.30 |

As provided, the center point formulation of Table 6 demonstrates long-term stability for the therapeutic antibody, even under stressed conditions of extended periods at high temperatures.

### In Vivo Tolerability Study

Assessment of injection-associated pain from subcutaneous injection of an aqueous pharmaceutical formulation of ixekizumab, at a high concentration (80mg/mL), is performed according to a study in which subjects receive a SQ injection of one of Formulation A or B (as provided in Table 8), followed by a SQ injection of the other of Formulation A or B some period of time (e.g., 1, 5, 7, 10, 14, etc., days) later. Subjects are then assessed for injection-associated pain based on the VAS scale scoring at specified time points (e.g., within 1 minute (i.e., immediately after injection), within 10 minutes, within 1 hour, within 4 hours within 1 day) after each injection.

**Table 8. Ixekizumab Pharmaceutical Formulation**

| **Formulation A** | | **Formulation B** | |
|---|---|---|---|
| (corresponds to center point formulation of Table 6) | | (commercial formulation of Taltz^{®}) | |
| ixekizumab | 80 mg/mL | ixekizumab | 80 mg/mL |
| pH | 5.7 | pH | 5.7 |
| PS-80 | 0.3mM | PS-80 | 0.03% w/v |
| sucrose | 80mM | NaCl | 200mM |
| ///////////////////////////////////////////////// | | citrate buffer | 20mM |

Accordingly, a single-dose, subject blinded, randomized, cross-over study is performed in which subjects are randomized into one of two treatment groups. Each treatment group receives subcutaneous injections of the pharmaceutical formulations comprising 80 mg/ml of ixekizumab, as set forth in Table 8, according to the following injection regimens.

Treatment group 1 receives a single dose of Formulation B, followed by a single dose of Formulation A seven days later. Treatment group 2 receives a single dose, by SQ injection, of Formulation A followed by a single dose, by SQ injection, of Formulation B fourteen days later. Injections are administered by medical personnel in the abdomen of the subject while the subject is in a sitting or reclining position. Subsequent injections may be alternated between abdominal quadrants. Assessment for injection-associated pain based on VAS scale scoring is performed immediately after each injection (e.g., within 1 min.) and at 10 minutes post injection. Results are provided in Tables 9 and 10 below.

**Table 9. Injection-Associated Pain Comparability Data**

| **Formulation** | **VAS Score** | **VAS Score** |
|---|---|---|
| | time post-injection (w/in 1 min.) | time post-injection (10 mins.) |
| **A** (N=63) | 3.52 | 0.68 |
| **B** (N=61) | 25.21 | 5.15 |

As shown in Table 9, Formulation A provides a substantial decrease in VAS score over Formulation B (the commercially available formulation of Taltz^{®}) both immediately after injection and at 10 minutes post-injection.

**Table 10. Patient Tolerability Analysis**

| **VAS Score** | **Formulation A** | **Formulation B** |
|---|---|---|
| | time post-injection | time post-injection |
| | (w/in 1 min.) | (w/in 1 min.) |
| **No pain** (VAS=0) | 26 (of 63 patients): 41.3% | 5 (of 61 patients): 8.2% |
| **Mild Pain** (VAS≤30) | 36 (of 63 patients): 57.1% | 36 (of 61 patients): 59.0% |
| **Moderate-to-Severe Pain** (VAS>30) | 1 (of 63 patients): 1.6% | 20 (of 61 patients): 32.8% |

As shown in Table 10, Formulation A provides a substantial improvement in patients experiencing no injection-associated pain immediately post-injection as well as a substantial benefit in the reduction of patients experiencing moderate-to-severe injection-associated pain immediately post-injection over Formulation B (the commercially available formulation of Taltz^{®}).

### In Vivo Pharmacokinetic Analysis

Pharmacokinetic analysis of an aqueous pharmaceutical formulation of ixekizumab may be performed according to a study in which subjects receive a SQ injection of one of Formulation A or B (as provided in Table 8). Subjects are then assessed for pharmacokinetic analysis at various time points (e.g., prior to SQ injection and then post-SQ injection such as 1-24 hrs., 1-90 days post-injection).

Accordingly, a single-dose, subject blinded, randomized, parallel design study is performed in which, on day 1, subjects are randomized into one of two treatment groups. Prior to receiving a treatment (e.g., day 1, pre-dose) a pre-dose sample from patients of both treatment groups is taken for pharmacokinetic property assessment. On Day 1, treatment group 1 receives a single, SQ injection of Formulation A and treatment group 2 receives a single, subcutaneous injection of Formulation B (as described in Table 8). Injections may be administered by medical personnel in the abdomen of the subjects. Post-dosing, samples are taken on study days 3, 5 (±1 day), 8 (±1 day), 11 (±1 day), 15 (±2 days), 22 (±2 days), 29 (±2 days), 43 (±2 days), 57 (±3 days), 71 (±3 days) and 85 (±3 days) to assess pharmacokinetic parameters including Cmax (maximum observed drug concentration), AUC[0-∞] (area under the concentrations versus time curve from time zero to infinity), AUC[0-tₗₐₛₜ] (area under the concentrations versus time curve from time zero on study Day 1 to time of last measurable concentration), and Tmax (time of the maximum observed drug concentration). Results are provided in Table 11.

**Table 11. In Vivo Pharmacokinetic Analysis**

| **Formulation** | **P_{K} Parameter** | **Value** | **Ratio** |
|---|---|---|---|
| | | (geometric least squares mean) | (Form. A / Form B) |
| Formulation A (N=33) | AUC[0-∞] (ug*day / mL) | 159 | 1.05 |
| Formulation B (N=32) | | 152 | |
| Formulation A (N=33) | AUC[0-tₗₐₛₜ] (ug*day / mL) | 153 | 1.04 |
| Formulation B (N=32) | | 146 | |
| Formulation A (N=33) | Cmax (ug/mL) | 6.29 | 1.00 |
| Formulation B (N=33) | | 6.31 | |
| Formulation A (N=33) | Tmax (days) | 4.09 | **(median of differences)** |
| Formulation B (N=33) | | 3.95 | |
| | | | 0 |

As shown in Table 11, Formulation A demonstrates comparable P_{K} parameters to Formulation B (the commercially available formulation of Taltz^{®}). Also, no severe adverse events were reported for either formulation and overall safety is consistent and comparable to Formulation B.

### Target Neutralization Assessment

Following incubation of samples of Formulation A at 5°C for 1, 6 and 12 months; 25°C for 1 month; and 35°C for 1 month, potency of Formulation A is assessed in comparison to Formulation B (of Table 8) by way of a cell-based bioassay. Briefly, murine osteoblast cell line MC3T3-E1, which endogenously expresses IL-17A receptor and stably expresses firefly luciferase gene, is cultured such that when IL-17A is present transcription of luciferase is induced at levels proportional to IL-17A activity. Previously incubated samples of Formulation A and B are introduced to culture wells of the cell-based bioassay, respectively, and following measurement of luciferase expression, inhibition dose curves are generated. Data is analyzed using a four parameter logistic curve fit. Relative potency is determined by calculating the ratio of the EC₅₀ for Formulation A in comparison to the EC₅₀ of Formulation B (e.g., the reference standard). Results are provided in Table 12.

**Table 12. Relative Potency Assessment of Formulation A (% relative to Formulation B)**

| **Incubation Period** | **Incubation Temp.** | | |
|---|---|---|---|
| | (°C) | | |
| (Months) | | | |
| | **5°C** | **25°C** | **35°C** |
| **1** | 101% | 98% | 101% |
| **6** | 103% | ND | ND |
| **12** | 98% | ND | ND |

As shown in Table 12, Formulation A demonstrates levels of target neutralization comparable to Formulation B (the commercially available formulation of Taltz^{®}) after extended periods of storage and under stressed conditions.

### Sequences

**SEQ ID NO: 1 (LCDR1 of Exemplary anti-IL17A antibody)**
   RSSRSLVHSRGNTYLH
**SEQ ID NO: 2 (LCDR2 of Exemplary anti-IL17A antibody)**
   KVSNRFI
**SEQ ID NO: 3 (LCDR3 of Exemplary anti-IL17A antibody)**
   SQSTHLPFT
**SEQ ID NO: 4 (HCDR1 of Exemplary anti-IL17A antibody)**
   GYSFTDYHIH
**SEQ ID NO: 5 (HCDR2 of Exemplary anti-IL17A antibody)**
   VINPMYGTTDYNQRFKG
**SEQ ID NO: 6 (HCDR3 of Exemplary anti-IL17A antibody)**
   YDYFTGTGVY
**SEQ ID NO: 7 (LCVR of Exemplary anti-IL17A antibody)** 
DIVMTQTPLSLSVTPGQPASISCRSSRSLVHSRGNTYLHWYLQKPGQSPQLLIY
KVSNRFIGVPDRFSGSGSGTDFTLKISRVEAEDVGVYYCSQSTHLPFTFGQGTK
LEIK
**SEQ ID NO: 8 (HCVR of Exemplary anti-IL17A antibody)** 
QVQLVQSGAEVKKPGSSVKVSCKASGYSFTDYHIHWVRQAPGQGLEWMGVIN
PMYGTTDYNQRFKGRVTITADESTSTAYMELSSLRSEDTAVYYCARYDYFTGT
GVYWGQGTLVTVSS
**SEQ ID NO: 9 (light chain of Exemplary anti-IL17A antibody)** 
DIVMTQTPLSLSVTPGQPASISCRSSRSLVHSRGNTYLHWYLQKPGQSPQLLIY
KVSNRF IGVPDRFSGSGSGTDFTLKISRVEAEDVGVYYCSQSTHLPFTFGQGT
KLEIKRTVAAPSVFIFPPSDEQLKSGTASVVCLLNNFYPREAKVQWKVDNALQ
SGNSQESVTEQDSKDSTYSLSSTLTLSKADYEKHKVYACEVTHQGLSSPVTKS
FNRG EC
**SEQ ID NO: 10 (heavy chain of Exemplary anti-IL17A antibody)**

QVQLVQSGAEVKKPGSSVKVSCKASGYSFTDYHIHWVRQAPGQGLEWMGVI
NPMYGTTDYNQRFKGRVTITADESTSTAYMELSSLRSEDTAVYYCARYDYFT
GTGVYWGQGTLVTVSSASTKGPSVFPLAPCSRSTSESTAALGCLVKDYFPEPV
TVSWNSGALTSGVHTFPAVLQSSGLYSLSSVVTVPSSSLGTKTYTCNVDHKPS
NTKV DKRVESKYGPPCPPCPAPEFLGGPSVFLFPPKPKDTLMISRTPEVTCVVV
DVSQEDPEVQFNWYVDGVEVHNAKTKPREEQFNSTYRVVSVLTVLHQDWLN
GKEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNV
FSCSVMHEALHNHYTQKSLSLSLG

## Claims

1. A bufferless aqueous pharmaceutical formulation comprising:
(i) an anti-IL-17A antibody at a concentration of 80 mg/mL +/- 10%;
(ii) sucrose in a concentration of 234mM +/- 10%; and
(iii) a surfactant in a concentration of between 0.005% w/v +/- 10% to 0.05% w/v +/-10%,
wherein, the pharmaceutical formulation is an aqueous solution at a pH between 5.2 to 6.5, the pharmaceutical formulation is substantially free of an ionic tonicity excipient and the anti-IL17A antibody comprises a light chain variable region (LCVR) and a heavy chain variable region (HCVR), wherein the LCVR comprises complementarity determining regions (CDRs) LCDR1, LCDR2, and LCDR3 and the HCVR comprises CDRs HCDR1, HCDR2, and HCDR3, wherein:
LCDR1 comprises the amino acid sequence of SEQ ID NO. 1,
LCDR2 comprises the amino acid sequence of SEQ ID NO. 2,
LCDR3 comprises the amino acid sequence of SEQ ID NO. 3,
HCDR1 comprises the amino acid sequence of SEQ ID NO. 4,
HCDR2 comprises the amino acid sequence of SEQ ID NO. 5, and
HCDR3 comprises the amino acid sequence of SEQ ID NO. 6.

2. An aqueous pharmaceutical formulation according to claim 1, wherein the surfactant is polysorbate 20 or polysorbate 80.

3. An aqueous pharmaceutical formulation according to claim 2, wherein the surfactant is polysorbate 80.

4. An aqueous pharmaceutical formulation according to any one of claims 1 to 3, wherein the pharmaceutical formulation is substantially free of L-amino acid excipients.

5. An aqueous pharmaceutical formulation according to any one of claims 1 to 4, wherein the LCVR comprises the amino acid sequence of SEQ ID NO. 7 and the HCVR comprises the amino acid sequence of SEQ ID NO. 8.

6. An aqueous pharmaceutical formulation according to claim 5, wherein anti-IL17A antibody comprises a light chain (LC) and a heavy chain (HC), wherein the LC comprises the amino acid sequence of SEQ ID NO. 9 and the HC comprises the amino acid sequence of SEQ ID NO. 10.

7. An aqueous pharmaceutical formulation according to any one of claims 1 to 6, wherein the anti-IL17A antibody is ixekizumab.

8. An aqueous pharmaceutical formulation according to any one of the preceding claims for use in the treatment of rheumatoid arthritis (RA), psoriasis (Ps), genital psoriasis (GenPs), pruritus, ankylosing spondylitis (AS), psoriatic arthritis (PA), palmoplantar pustulosis (PPP), hidradenitis suppurativa (HS) or multiple myeloma (MM).

9. An aqueous pharmaceutical formulation for use according to claim 8, wherein the pharmaceutical formulation is administered subcutaneously to a patient at an initial dose on day 0, followed by a subcutaneous administration of the pharmaceutical formulation to the patient at every four week interval thereafter,
wherein the pharmaceutical formulation administered to the patient at every four week interval after the initial dose comprises the anti-IL17A antibody at a concentration of about 80 mg/mL.

10. An aqueous pharmaceutical formulation for use according to claim 8, wherein the pharmaceutical formulation is administered subcutaneously to a patient at an initial dose on day 0, followed by a subcutaneous administration of the pharmaceutical formulation to the patient at every two week interval thereafter,
wherein the pharmaceutical formulation administered to the patient at every two week interval after the initial dose comprises the anti-IL17A antibody at a concentration of about 80 mg/mL.

11. An aqueous pharmaceutical formulation for use according to claim 8, wherein the pharmaceutical formulation is
administered subcutaneously to a patient at an initial dose on day 0, followed by a subcutaneous administration of the pharmaceutical formulation to the patient on each of days 14, 28, 42, 56, 70 and 84, and followed by a subcutaneous administration of the pharmaceutical formulation to the patient at every four week interval thereafter, wherein the pharmaceutical formulation, administered to the patient at each of days 14, 28, 42, 56, 70 and 84, and every four week interval thereafter, comprises the anti-IL17A antibody at a concentration of about 80 mg/mL.

12. An aqueous pharmaceutical formulation for use according to any one of claims 9 to 11, wherein the initial dose of the pharmaceutical formulation comprises about 160 mg of the anti-IL17A antibody.

13. An aqueous pharmaceutical formulation for use according to claim 12, wherein the about 160 mg initial dose of the pharmaceutical formulation comprises two doses of the pharmaceutical formulation, each dose comprising about 80 mg of the anti-IL17A antibody.

14. An aqueous pharmaceutical formulation according to any one of claims 1 to 7 for use in the reduction of injection-associated pain experienced by a patient at the time of, or shortly after, subcutaneous, intraperitoneal and/or intramuscular administration of the aqueous pharmaceutical formulation comprising an anti-IL17A antibody.

15. An aqueous pharmaceutical formulation for use according to claim 14, wherein the injection-associated pain experienced by a patient comprises a VAS score of less than 30 mm or less than 20 mm.

16. An aqueous pharmaceutical formulation for use according to claim 14 or claim 15, wherein the anti-IL17A antibody is ixekizumab.

17. An aqueous pharmaceutical formulation for use according to claim 8, wherein the pharmaceutical formulation is bufferless, substantially free of L-amino acid excipients and the surfactant is polysorbate 80.

## Patentansprüche

1. Pufferfreie wässrige pharmazeutische Formulierung, umfassend:
(i) ein Anti-IL-17A-Antikörper in einer Konzentration von 80 mg/ml +/- 10 %;
(ii) Saccharose in einer Konzentration von 234 mM +/- 10 %; und
(iii) ein Tensid in einer Konzentration zwischen 0,005 Gew.-/Vol.-% +/- 10 % und 0,05 Gew.-/Vol.-% +/- 10 %,
wobei die pharmazeutische Formulierung eine wässrige Lösung bei einem pH-Wert zwischen 5,2 und 6,5 ist, die pharmazeutische Formulierung im Wesentlichen frei von einem ionischen Tonizitätshilfsstoff ist und der Anti-IL17A-Antikörper eine variable Region der leichten Kette (LCVR) und eine variable Region der schweren Kette (HCVR) umfasst, wobei die LCVR die komplementaritätsbestimmenden Regionen (CDRs) LCDR1, LCDR2 und LCDR3 umfasst und die HCVR die CDRs HCDR1, HCDR2 und HCDR3 umfasst, wobei:
LCDR1 die Aminosäuresequenz von SEQ ID NO: 1 umfasst,
LCDR2 die Aminosäuresequenz von SEQ ID NO: 2 umfasst,
LCDR3 die Aminosäuresequenz von SEQ ID NO: 3 umfasst,
HCDR1 die Aminosäuresequenz von SEQ ID NO: 4 umfasst,
HCDR2 die Aminosäuresequenz von SEQ ID NO: 5 umfasst und
HCDR3 die Aminosäuresequenz von SEQ ID NO: 6 umfasst.

2. Wässrige pharmazeutische Formulierung nach Anspruch 1, wobei das Tensid Polysorbat 20 oder Polysorbat 80 ist.

3. Wässrige pharmazeutische Formulierung nach Anspruch 2, wobei das Tensid Polysorbat 80 ist.

4. Wässrige pharmazeutische Formulierung nach einem der Ansprüche 1 bis 3, wobei die pharmazeutische Formulierung im Wesentlichen frei von L-Aminosäure-Hilfsstoffen ist.

5. Wässrige pharmazeutische Formulierung nach einem der Ansprüche 1 bis 4, wobei die LCVR die Aminosäuresequenz von SEQ ID NO: 7 umfasst und die HCVR die Aminosäuresequenz von SEQ ID NO: 8 umfasst.

6. Wässrige pharmazeutische Formulierung nach Anspruch 5, wobei der Anti-IL17A-Antikörper eine leichte Kette (LC) und eine schwere Kette (HC) umfasst, wobei die LC die Aminosäuresequenz von SEQ ID NO: 9 umfasst und die HC die Aminosäuresequenz von SEQ ID NO: 10 umfasst.

7. Wässrige pharmazeutische Formulierung nach einem der Ansprüche 1 bis 6, wobei der Anti-IL17A-Antikörper Ixekizumab ist.

8. Wässrige pharmazeutische Formulierung nach einem der vorstehenden Ansprüche zur Verwendung bei der Behandlung von rheumatoider Arthritis (RA), Psoriasis (Ps), genitaler Psoriasis (GenPs), Pruritus, Spondylitis ankylosans (AS), Psoriasis-Arthritis (PA), Pustulosis palmoplantaris (PPP), Hidradenitis suppurativa (HS) oder multiplem Myelom (MM).

9. Wässrige pharmazeutische Formulierung zur Verwendung nach Anspruch 8, wobei die pharmazeutische Formulierung einem Patienten in einer Anfangsdosis an Tag 0 subkutan verabreicht wird, gefolgt von einer subkutanen Verabreichung der pharmazeutischen Formulierung an den Patienten in vierwöchigen Intervallen danach,
wobei die pharmazeutische Formulierung, die dem Patienten in vierwöchigen Intervallen nach der Anfangsdosis verabreicht wird, den Anti-IL17A-Antikörper in einer Konzentration von etwa 80 mg/ml umfasst.

10. Wässrige pharmazeutische Formulierung zur Verwendung nach Anspruch 8, wobei die pharmazeutische Formulierung einem Patienten in einer Anfangsdosis an Tag 0 subkutan verabreicht wird, gefolgt von einer subkutanen Verabreichung der pharmazeutischen Formulierung an den Patienten in zweiwöchigen Intervallen danach,
wobei die pharmazeutische Formulierung, die dem Patienten in zweiwöchigen Intervallen nach der Anfangsdosis verabreicht wird, den Anti-IL17A-Antikörper in einer Konzentration von etwa 80 mg/ml umfasst.

11. Wässrige pharmazeutische Formulierung zur Verwendung nach Anspruch 8, wobei die pharmazeutische Formulierung einem Patienten subkutan in einer Anfangsdosis an Tag 0 verabreicht wird, gefolgt von einer subkutanen Verabreichung der pharmazeutischen Formulierung an den Patienten an jedem der Tage 14, 28, 42, 56, 70 und 84, und gefolgt von einer subkutanen Verabreichung der pharmazeutischen Formulierung an den Patienten in vierwöchigen Intervallen danach, wobei die pharmazeutische Formulierung, die dem Patienten an jedem der Tage 14, 28, 42, 56, 70 und 84 und in vierwöchigen Intervallen danach verabreicht wird, den Anti-IL17A-Antikörper in einer Konzentration von etwa 80 mg/ml umfasst.

12. Wässrige pharmazeutische Formulierung zur Verwendung nach einem der Ansprüche 9 bis 11, wobei die Anfangsdosis der pharmazeutischen Formulierung etwa 160 mg des Anti-IL17A-Antikörpers umfasst.

13. Wässrige pharmazeutische Formulierung zur Verwendung nach Anspruch 12, wobei die Anfangsdosis von etwa 160 mg der pharmazeutischen Formulierung zwei Dosen der pharmazeutischen Formulierung umfasst, wobei jede Dosis etwa 80 mg des Anti-IL17A-Antikörpers umfasst.

14. Wässrige pharmazeutische Formulierung nach einem der Ansprüche 1 bis 7 zur Verwendung bei der Verringerung von injektionsassoziiertem Schmerz, der von einem Patienten zum Zeitpunkt der oder kurz nach der subkutanen, intraperitonealen und/oder intramuskulären Verabreichung der wässrigen pharmazeutischen Formulierung, die einen Anti-IL17A-Antikörper umfasst, empfunden wird.

15. Wässrige pharmazeutische Formulierung zur Verwendung nach Anspruch 14, wobei der von einem Patienten erfahrene injektionsassoziierte Schmerz einen VAS-Wert von weniger als 30 mm oder weniger als 20 mm umfasst.

16. Wässrige pharmazeutische Formulierung zur Verwendung nach Anspruch 14 oder 15, wobei der Anti-IL17A-Antikörper Ixekizumab ist.

17. Wässrige pharmazeutische Formulierung zur Verwendung nach Anspruch 8, wobei die pharmazeutische Formulierung pufferfrei und im Wesentlichen frei von L-Aminosäure-Hilfsstoffen ist und das Tensid Polysorbat 80 ist.

## Revendications

1. Formulation pharmaceutique aqueuse sans tampon comprenant :
(i) un anticorps anti-IL-17A à une concentration de 80 mg/mL +/- 10 % ;
(ii) du saccharose en une concentration de 234 mM +/- 10 % ; et
(iii) un agent tensioactif en une concentration comprise de 0,005 % en poids/volume +/- 10 % à 0,05 % en poids/volume +/- 10 %,
dans laquelle, la formulation pharmaceutique est une solution aqueuse à un pH compris entre 5,2 et 6,5, la formulation pharmaceutique est sensiblement dépourvue d'un excipient de tonicité ionique et l'anticorps anti-IL-17A comprend une région variable de chaîne légère (LCVR) et une région variable de chaîne lourde (HCVR), dans laquelle la LCVR comprend des régions de détermination de complémentarité (CDR) LCDR1, LCDR2 et LCDR3, et la HCVR comprend des CDR HCDR1, HCDR2 et HCDR3, dans laquelle :
LCDR1 comprend la séquence d'acides aminés de SEQ ID NO. 1,
LCDR2 comprend la séquence d'acides aminés de SEQ ID NO. 2,
LCDR3 comprend la séquence d'acides aminés de SEQ ID NO. 3,
HCDR1 comprend la séquence d'acides aminés de SEQ ID NO. 4,
HCDR2 comprend la séquence d'acides aminés de SEQ ID NO. 5, et
HCDR3 comprend la séquence d'acides aminés de SEQ ID NO. 6.

2. Formulation pharmaceutique aqueuse selon la revendication 1, dans laquelle l'agent tensioactif est du polysorbate 20 ou du polysorbate 80.

3. Formulation pharmaceutique aqueuse selon la revendication 2, dans laquelle l'agent tensioactif est du polysorbate 80.

4. Formulation pharmaceutique aqueuse selon l'une quelconque des revendications 1 à 3, dans laquelle la formulation pharmaceutique est sensiblement dépourvue d'excipients acides L-aminés.

5. Formulation pharmaceutique aqueuse selon l'une quelconque des revendications 1 à 4, dans laquelle la LCVR comprend la séquence d'acides aminés de SEQ ID NO. 7 et la HCVR comprend la séquence d'acides aminés de SEQ ID NO. 8.

6. Formulation pharmaceutique aqueuse selon la revendication 5, dans laquelle l'anticorps anti-IL-17A comprend une chaîne légère (LC) et une chaîne lourde (HC), dans laquelle la LC comprend la séquence d'acides aminés de SEQ ID NO. 9 et la HC comprend la séquence d'acides aminés de SEQ ID NO. 10.

7. Formulation pharmaceutique aqueuse selon l'une quelconque des revendications 1 à 6, dans laquelle l'anticorps anti-IL-17A est de l'ixékizumab.

8. Formulation pharmaceutique aqueuse selon l'une quelconque des revendications précédentes pour utilisation dans le traitement de polyarthrite rhumatoïde (RA), de psoriasis (Ps), de psoriasis génital (GenPs), de prurit, de spondylarthrite ankylosante (AS), d'arthrite psoriasique (PA), de pustulose palmo-plantaire (PPP), d'hidradenitis suppurativa (HS) ou de myélome multiple (MM).

9. Formulation pharmaceutique aqueuse pour utilisation selon la revendication 8, dans laquelle la formulation pharmaceutique est administrée par voie sous-cutanée à un patient à une dose initiale au jour 0, suivie par une administration sous-cutanée de la formulation pharmaceutique au patient à un intervalle de toutes les quatre semaines par la suite,
dans laquelle la formulation pharmaceutique administrée au patient à un intervalle de toutes les quatre semaines après la dose initiale comprend l'anticorps anti-IL-17A à une concentration d'environ 80 mg/mL.

10. Formulation pharmaceutique aqueuse pour utilisation selon la revendication 8, dans laquelle la formulation pharmaceutique est administrée par voie sous-cutanée à un patient à une dose initiale au jour 0, suivie par une administration sous-cutanée de la formulation pharmaceutique au patient à un intervalle de toutes les deux semaines par la suite,
dans laquelle la formulation pharmaceutique administrée au patient à un intervalle de toutes les deux semaines après la dose initiale comprend l'anticorps anti-IL-17A à une concentration d'environ 80 mg/mL.

11. Formulation pharmaceutique aqueuse pour utilisation selon la revendication 8, dans laquelle la formulation pharmaceutique est
administrée par voie sous-cutanée à un patient à une dose initiale au jour 0, suivie par une administration sous-cutanée de la formulation pharmaceutique au patient à chacun des jours 14, 28, 42, 56, 70 et 84, et suivie par une administration sous-cutanée de la formulation pharmaceutique au patient à un intervalle de toutes les quatre semaines par la suite, dans laquelle la formulation pharmaceutique, administrée au patient à chacun des jours 14, 28, 42, 56, 70 et 84, et à un intervalle de toutes les quatre semaines par la suite, comprend l'anticorps anti-IL-17A à une concentration d'environ 80 mg/mL.

12. Formulation pharmaceutique aqueuse pour utilisation selon l'une quelconque des revendications 9 à 11, dans laquelle la dose initiale de la formulation pharmaceutique comprend environ 160 mg de l'anticorps anti-IL-17A.

13. Formulation pharmaceutique aqueuse pour utilisation selon la revendication 12, dans laquelle la dose initiale d'environ 160 mg de la formulation pharmaceutique comprend deux doses de la formulation pharmaceutique, chaque dose comprenant environ 80 mg de l'anticorps anti-IL-17A.

14. Formulation pharmaceutique aqueuse selon l'une quelconque des revendications 1 à 7 pour utilisation dans la réduction de douleur associée à l'injection, ressentie par un patient au moment de, ou peu de temps après, une administration sous-cutanée, intrapéritonéale et/ou intramusculaire de la formulation pharmaceutique aqueuse comprenant un anticorps anti-IL-17A.

15. Formulation pharmaceutique aqueuse pour utilisation selon la revendication 14, dans laquelle la douleur associée à l'injection, ressentie par un patient comprend un score EVA inférieur à 30 mm ou inférieur à 20 mm.

16. Formulation pharmaceutique aqueuse pour utilisation selon la revendication 14 ou la revendication 15, dans laquelle l'anticorps anti-IL-17A est de l'ixékizumab.

17. Formulation pharmaceutique aqueuse pour utilisation selon la revendication 8, dans laquelle la formulation pharmaceutique est sans tampon, sensiblement dépourvue d'excipients acides L-aminés et l'agent tensioactif est du polysorbate 80.
